# EUROPEAN PATENT SPECIFICATION

(11) **EP 4 000 530 B1**
(45) Date of publication and mention of the grant of the patent: **30.09.2026**
(21) Application number: 19938298.7
(22) Date of filing: 27.11.2019
(51) Int. Cl.: A61B 8/08

(54) **MEASUREMENT METHOD AND SYSTEM FOR OBTAINING ARTERIOSCLEROSIS INDEX BASED ON ULTRASOUND DEVICE**
MESSVERFAHREN UND SYSTEM ZUR BESTIMMUNG DES ARTERIOSKLEROSEINDEX AUF BASIS EINER ULTRASCHALLVORRICHTUNG
PROCÉDÉ ET SYSTÈME DE MESURE POUR OBTENIR UN INDICE D'ARTÉRIOSCLÉROSE BASÉ SUR UN DISPOSITIF À ULTRASONS

(30) Priority: 19.07.2019 CN 201910654940
(43) Date of publication of application: 25.05.2022
(73) Proprietor: Vinno Technology (Suzhou) Co., Ltd., Jiangsu 215123 (CN)
(72) Inventor: ZHAO, Yiming, Suzhou Industrial Park, Jiangsu 215123 (CN); ZHANG, Hongbing, Suzhou Industrial Park, Jiangsu 215123 (CN); CAO, Zexu, Suzhou Industrial Park, Jiangsu 215123 (CN); GUO, Dongmei, Suzhou Industrial Park, Jiangsu 215123 (CN)
(74) Representative: Arnold & Siedsma
(86) International application number: PCT/CN2019/121367
(87) International publication number: WO 2021/012561

(56) References cited:
- CN-A- 102 170 821
- CN-A- 103 140 165
- CN-A- 103 989 462
- CN-A- 106 798 547
- CN-A- 106 798 547
- CN-A- 106 798 547
- CN-A- 109 730 723
- CN-A- 110 652 318
- CN-A- 110 881 967
- JP-A- H06 261 899
- US-A1- 2002 091 328
- US-A1- 2007 016 083
- US-A1- 2021 369 235
- SAFAR MICHEL E.: "Arterial stiffness as a risk factor for clinical hypertension", vol. 15, no. 2, 12 October 2017 (2017-10-12), GB, pages 97 - 105, XP093058300, ISSN: 1759-5002, Retrieved from the Internet <URL:http://www.nature.com/articles/nrcardio.2017.155> DOI: 10.1038/nrcardio.2017.155
- SZMIGIELSKI C ET AL: "Pulse wave velocity correlates with aortic atherosclerosis assessed with transesophageal echocardiography", JOURNAL OF HUMAN HYPERTENSION, vol. 30, no. 2, 1 January 2016 (2016-01-01), pages 90 - 94, XP037323575, ISSN: 0950-9240, DOI: 10.1038/JHH.2015.35

## Description

### TECHNICAL FIELD

The present application relates to the field of medical ultrasound technology, and particularly to a measurement method and system for obtaining an arteriosclerosis index based on an ultrasound device.

### BACKGROUND

The cardiovascular disease is a major disease that seriously endangers human health. Arteriosclerosis is the common pathophysiological basis of most cardiovascular diseases. Arteriosclerosis of any body part may cause an ischemic function in a corresponding area, such as the coronary heart disease, cerebral stroke, etc. Therefore, knowing the degree of arterial stiffness is the key to the prevention and treatment of the cardiovascular diseases.

The current arteriosclerosis detection device is mainly summarized as two types: an invasive detection device and a non-invasive detection device. The invasive detection device detects the degree of the arteriosclerosis by using the arteriography. However, the detection device based on the arteriography has an invasive operation, and has characteristics of a complicated detection, a high price and a low sensitivity. The non-invasive detection device can apply methods such as vascular compliance detection based on the ultrasound imaging, which also have problems such as a high cost, a complicated operation, needing for professional guidance, and no conduciveness to portable monitoring, etc.

The document JPH06261899A discloses an ultrasonic system including a means for measuring a signal phase difference at the reception time adjacent signal conversion unit including means for performing moving average processing of the signal phase difference, or the signal phase difference in adjacent reception time measurement, signals and means for measuring further difference of the phase difference, means or calculating a conjugate product of the complex signal in the signal, the signal conversion unit includes means for performing moving average processing on the difference of the phase difference or adjacent reception time, a signal converter including a means for calculating the complex moving average of the conjugate product.

The document "Safar Michel E.: 'Arterial stiffness as a risk factor for clinical hypertension', Nature Reviews Cardiology, vol. 15, no. 2, 12 October 2017, pages 97-105, XP093058300" discloses schematic measurements of arterial stiffness and distensibility in hypertensive and normotensive settings. These measurements, deduced from the classical Bramwell and Hill formula, are made between two distinct carotid and femoral arterial sites. Pulse wave velocity, PWV, and distensibility can be calculated according to the distance L between the two sites, and the time interval ΔT between the aortic and femoral pressure waves.

The document CN 109730723A discloses a method for determining pulse transmission time, arteriosclerosis detection device and system. The method comprises the following steps of: receiving single-conduction electrocardiosignals; receiving pulse wave signals of at least one body part, wherein pulse wave signals are detected by a micro ultrasonic module arranged at the corresponding body part; determining the pulse transmission time with the R wave of single-conduction electrocardiosignals as the starting point and with the characteristic point of pulse wave signals of at least one body part as the ending point.

### SUMMARY

The present application provides a measurement method and system for obtaining an arteriosclerosis index based on an ultrasound device.

In order to implement one of the above purposes of the present application, a measurement method for obtaining an arteriosclerosis index based on an ultrasound device is provided in an embodiment, which includes: acquiring two measurement points *P1* and *P2* at different measurement positions at random, recording an extension distance L between the two measurement points *P1* and *P2*, acquiring a measurement time difference *ΔT* between the two measurement points by using an R peaks of an ECG signal as a time synchronization signal;
acquiring a pulse wave velocity (PWV) according to the extension distance *L* and the measurement time difference *ΔT,* wherein *PWV=L*/*ΔT*;
acquiring the arteriosclerosis index *D* according to the PWV, wherein *D*=(3.57/PWV)²;
the acquiring the measurement time difference *ΔT* between the two measurement points by using the *R* peaks of an ECG signal as a time synchronization signal specifically includes:
   acquiring an oscillogram at each measurement point, and selecting at least one characteristic point in the oscillogram corresponding to the each measurement point;
   acquiring a time difference between each characteristic point and the *R* peaks of the ECG signal;
   obtaining the *ΔT* according to a plurality of time difference obtained between a plurality of characteristic points and the *R* peaks of the ECG signal;
   the method further includes:
   identifying an oscillogram corresponding to the measurement point *P1* as a first oscillogram, and identifying an oscillogram corresponding to the measurement point *P2* as a second oscillogram;
   identifying a characteristic point selected in the first oscillogram as a characteristic point *X*, and identifying a characteristic point selected in the second oscillogram as a characteristic point *Y*;
   identifying a difference value between each characteristic point *X* and the *R* peaks of the ECG signal selected according to a same regulation as *ΔX,* and identifying a difference value between each characteristic point *Y* and the *R* peaks of the ECG signal selected according to the same regulation as *ΔY;*
   calculating an average value *ΔX̅* of *ΔX* and an average value Δ*Y̅* of *ΔY,* and obtaining Δ*T* = |Δ*X̅* - Δ*Y̅*|;
   the method further includes:
      determining whether an absolute value of a difference value between each *ΔX* and *ΔX̅* is greater than a first preset threshold, determining that a current *ΔX* is invalid if the absolute value of the difference value between each *ΔX* and *ΔX̅* is greater than the first preset threshold, recalculating the *ΔX̅* after excluding the invalid *ΔX,* and ending the calculation when determining that the absolute value of the difference value between each *ΔX* and the *ΔX̅* obtained by recalculating is not greater than the first preset threshold;
      determining whether an absolute value of a difference value between each *ΔY* and *ΔY̅* is greater than a second preset threshold, determining that a current *ΔY* is invalid if the absolute value of the difference value between each *ΔY* and *ΔY̅* is greater than the second preset threshold, recalculating the Δ*Y̅* after excluding the invalid *ΔY*, and ending the calculation when determining that the absolute value of the difference value between each Δ*Y* and the Δ*Y̅* obtained by recalculating is not greater than the second preset threshold.

As an improvement according to an embodiment of the present application, "the selecting at least one characteristic point in the oscillogram corresponding to the each measurement point" specifically includes:
searching for at least one maximum first derivative *M* in a current oscillogram, searching forward a first point *P3* with a first derivative greater than *k1*M* from a diastolic end of the current first derivative *M,* searching backward a first point *P4* with a first derivative greater than *k2*M* from the point *P3,* and selecting the point *P4* as the characteristic point of the current oscillogram, wherein *0<k2<k1<1;*
   or
searching for at least one point *P5* with a maximum first derivative in the current oscillogram, making a tangent at the point *P5* and identifying the tangent as a systolic tangent, searching for a lowest point *P6* of a diastole in a period corresponding to the point *P5,* making a tangent at the point *P6* and identifying the tangent as a diastolic tangent, calculating an intersection point *P7* of the systolic tangent and diastolic tangent, and selecting the intersection point *P7* as the characteristic point of the current oscillogram.

In order to implement one of the above purposes of the present application, a measurement system for obtaining an arteriosclerosis index based on an ultrasound device is provided in an embodiment, which includes: a first measurement module, configured to acquire two measurement points *P1* and *P2* at different measurement positions at random, and record an extension distance *L1* between the two measurement points *P1* and *P2*;
a second measurement module, configured to obtain a measurement time difference *ΔT* between the two measurement points by using an *R* peaks of an ECG signal as a time synchronization signal;
a processing module, configured to obtain a pulse wave velocity (PWV) according to the extension distance *L*and the measurement time difference *ΔT,* wherein *PWV=L*/Δ*T,* and acquiring the arteriosclerosis index *D* according to the pulse wave velocity (PWV), wherein *D*=(3.57/PWV)²;
the second measurement module is specifically configured to: acquire an oscillogram at each measurement point, select at least one characteristic point in the oscillogram corresponding to each measurement point;
acquire a time difference between each characteristic point and the *R* peaks of the ECG signal; and
obtain *ΔT* according to a plurality of time differences obtained between a plurality of characteristic points and the *R* peaks of the ECG signal;
the second measurement module is specifically configured to: identify an oscillogram corresponding to the measurement point *P1* as a first oscillogram, identify an oscillogram corresponding to the measurement point *P2* as a second oscillogram;
identify a characteristic point selected in the first oscillogram as a characteristic point *X*, identify a characteristic point selected in the second oscillogram as a characteristic point *Y*;
identify a difference value between each characteristic point *X* and an *R* peaks of the ECG signal selected according to a same regulation as *ΔX,* identify a difference value between each characteristic point *Y* and the *R* peaks of the ECG signal selected according to the same regulation as *ΔY;*
calculate an average value *ΔX̅* of the *ΔX* and an average value Δ*Y̅* of the *ΔY,* and obtain *ΔT* = |Δ*X̅* - Δ*Y̅*|;
the system further includes a screening module which is configured to: determine whether an absolute value of a difference value between each *ΔX* and *ΔX̅* is greater than a first preset threshold, determine that a current *ΔX* is invalid if the absolute value of the difference value between each *ΔX* and *ΔX̅* is greater than the first preset threshold, recalculate the Δ*X̅* after excluding the invalid *ΔX,* and end the calculation when determining that the absolute value of the difference value between each ΔX and the Δ*X̅* obtained by recalculating is not greater than the first preset threshold;
determine whether an absolute value of a difference value between each *ΔY* and Δ*Y̅* is greater than the second preset threshold, determine that a current *ΔY* is invalid when the absolute value of the difference value between each *ΔY* and the Δ*Y̅* is greater than the second preset threshold, recalculate the Δ*Y̅* after excluding the invalid *ΔY,* and end the calculation when determining that the absolute value of the difference value between each *ΔY* and the Δ*Y̅* obtained by recalculating is not greater than the second preset threshold.

As an improvement according to an embodiment of the present application, the second measurement module is configured to, when selecting at least one characteristic point in the oscillogram corresponding to each measurement point: search for at least one maximum first derivative *M* in a current oscillogram, search forward a first point *P3* with a first derivative greater than *k1*M* from a diastolic end of the current first derivative *M,* search backward a first point *P4* with a first derivative greater than *k2*M* from the point *P3,* and select the point *P4* as the characteristic point of the current oscillogram, wherein *0<k2<k1<1;*
or
search for at least one point *P5* with a maximum first derivative in the current oscillogram, make a tangent at the point *P5* and identify the tangent as a systolic tangent, search for a lowest point *P6* of a diastole in a period corresponding to the point *P5,* make a tangent at the point *P6* and identify the tangent as a diastolic tangent, calculate an intersection point *P7* of the systolic tangent and diastolic tangent, and select the intersection point *P7* as the characteristic point of the current oscillogram.

Compared to the prior art, the beneficial effects of the present application is that: in the measurement method and system for obtaining the arteriosclerosis index based on the ultrasound device in the present application, the *R* peaks of the ECG signal is used as a time synchronization signal to calculate the measurement time difference between two measurement points to obtain the arteriosclerosis index, which can improve the calculation accuracy and reduce the measurement cost without introducing other devices.

### BRIEF DESCRIPTION OF THE DRAWINGS

FIG. 1 is a flow chart showing a measurement method for obtaining an arteriosclerosis index based on an ultrasound device according to an embodiment of the present application.
FIG. 2 is a schematic structure diagram of a corresponding relationship between an ECG signal and a waveform diagram according to a specific example of the present application.
FIGS. 3 and 4 are schematic diagrams of oscillograms involved in different examples of the present application.
FIG. 5 is a schematic diagram of combining different blood vessel oscillograms and ECG signals according to a specific example of the present application.
FIG. 6 is a schematic diagram of modules of a measurement system for obtaining an arteriosclerosis index based on an ultrasound device according to an embodiment of the present application.

### DETAILED DESCRIPTION

The present application will be described in detail with reference to the specific embodiments shown in the accompanying drawings. However, these embodiments do not limit the present application, and transformations in the structure, method, or function made by those skilled in the art based on these embodiments all fall into the protection scope of the present application.

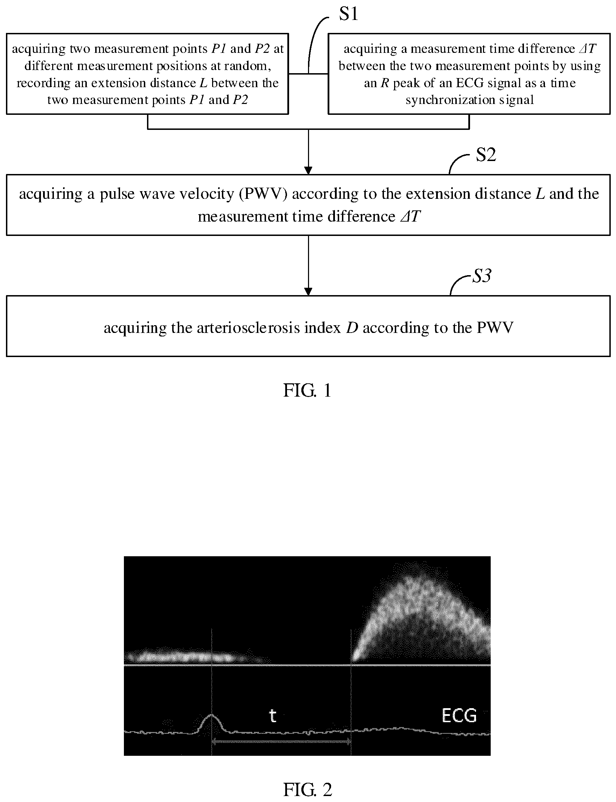

In an embodiment of the present application, as shown in FIG. 1, a measurement method for obtaining an arteriosclerosis index based on an ultrasound device is provided. The method includes following steps. S1: two measurement points *P1* and *P2* at different measurement positions are arbitrarily acquired, and an extension distance *L1* between the points *P1* and *P2* is recorded; an R peaks of an ECG signal is used as a time synchronization signal to acquire a measurement time difference *ΔT* between the two measurement points. S2: a pulse wave propagation velocity *PWV* is acquired according to the extension distance *L1* and the measurement time difference *ΔT,* with *PWV=L*/*ΔT.* S3: the arteriosclerosis index D is acquired according to the pulse wave propagation velocity *PWV,* with *D=(3.57*/*PWV)².*

For the step S1, different measurement positions are usually two blood vessel positions which are far apart. In the specific embodiment of the present application, the two blood vessel positions can be selected as the carotid artery and the femoral artery respectively; and the two measurement points *P1* and *P2* are any point respectively on the carotid artery and the femoral artery.

After the positions of the two measurement points are selected, the extension distance between the two measurement points can be obtained in many ways. The manner for obtaining the extension distance *L* is the prior art, and will not be further described here. It should be noted that the extension distance *L* is a physical extension distance formed by a flow path of blood from one measurement point to another measurement point.

The ECG signal is the abbreviation of electrocardiogram, and the Chinese meaning is an electrocardiosignal. For the same user, the ECG signal is fixed. In such a way, by using the R peaks of the ECG signal as the time synchronization signal, it can be ensured that the obtained time difference *ΔT* is more accurate, and there is no need to introduce other instruments to obtain other reference variables and the purpose of obtaining the measurement time difference *ΔT* can be achieved.

In an embodiment of the present application, step S1 of "using the R peaks of the ECG signal as a time synchronization signal to obtain the measurement time difference *ΔT* between two measurement points" specifically includes: at S11, an oscillogram at each measurement point is acquired, and at least one characteristic point in the oscillogram corresponding to each measurement point is selected; at S12, a time difference between each characteristic point and the *R* peaks of the ECG signal is acquired; at S13, *ΔT* is obtained according to the time differences between the obtained multiple characteristic points and the *R* peaks of the ECG signal.

As shown in FIG. 2, in this example, the time difference between the R peaks of the ECG signal and the selected characteristic point in the oscillogram corresponding to one measurement point is denoted by *t*.

Correspondingly, in this embodiment, the method specifically includes: the oscillogram corresponding to the measurement point *P1* is identified as a first oscillogram, and the oscillogram corresponding to the measurement point *P2* is identified as a second oscillogram; the characteristic point selected in the first oscillogram is identified by a characteristic point *X*, and the characteristic point selected in the second oscillogram is identified by a characteristic point *Y*; a difference value between each characteristic point X and the R peaks of the ECG signal selected according to the same regulation is identified by *ΔX,* and a difference value between each characteristic point *Y* and the *R* peaks of the ECG signal selected according to the same regulation is identified by *ΔY*; an average value Δ*X̅* of Δ*X̅* and an average value Δ*Y̅* of ΔY are calculated, and Δ*T* = |Δ*X̅* - Δ*̅Y̅*̅|̅.

It should be noted that if the number of the selected characteristic identifiers in the first oscillogram and the second oscillogram is one respectively, there is no need to calculate Δ*X̅* and Δ*Y̅*, and the *ΔT* can be directly obtained based on the Δ*X̅* and Δ*Y* corresponding to each characteristic point, that is, when the number of the selected characteristic identifiers in the two oscillograms is respectively one, it is satisfied that *ΔT*=|*ΔX-ΔY*|*.*

According to the claimed invention, when a plurality of characteristic points are selected in each oscillogram, abnormal parameters may exist in the plurality of characteristic points. In the preferred embodiment of the present application, in order to ensure the accuracy of calculation, the method further includes: it is determined whether an absolute value of the difference value between each *ΔX* and Δ*X̅* is greater than a first preset threshold, if yes, it is determined that the current *ΔX* is invalid, then the invalid *ΔX* is excluded and Δ*X̅* is recalculated, and when it is determined that the absolute value of the difference value between each *ΔX* and Δ*X̅* obtained by recalculating is not greater than the first preset threshold, the calculation ends; it is determined whether an absolute value of the difference value between each *ΔY* and Δ*Y̅* is greater than the second preset threshold, if yes, it is determined that the current *ΔY* is invalid, then the invalid *ΔY* is excluded and Δ*Y̅* is recalculated, and when it is confirmed that the absolute value of the difference value between each *ΔY* and *ΔY̅* obtained by recalculating is not greater than the second preset threshold, the calculation ends.

The first preset threshold and the second preset threshold are both preset known parameter values, and magnitudes of which are related to the heart rate signal, for example, 0.1s, which will not be further described here.

In the implementation mode of the present application, there are two modes for selecting the characteristic points.

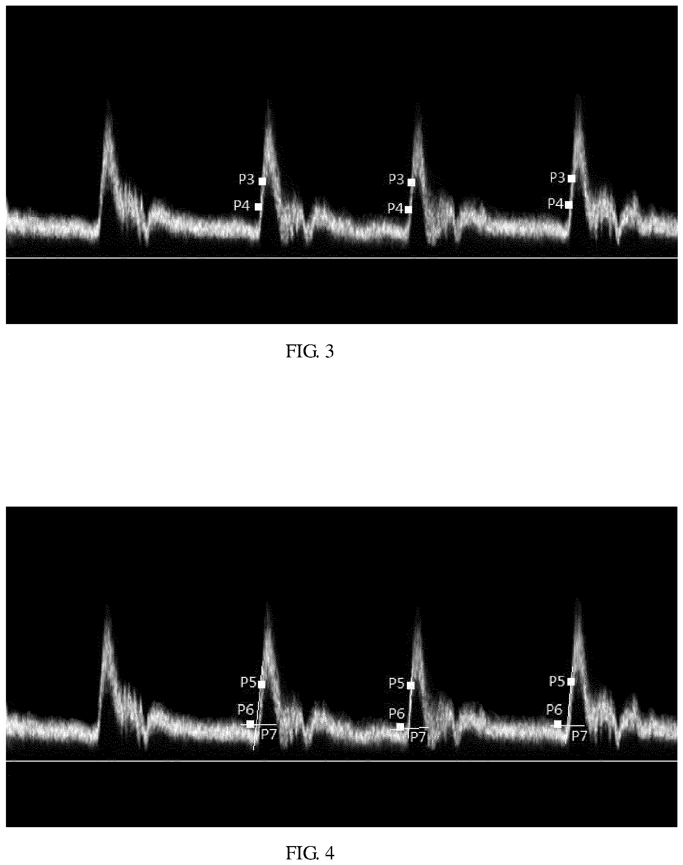

In a first implementation mode, the characteristic point in each oscillogram is obtained by using a derivative threshold method. Referring to FIG. 3, in this embodiment, the method specifically includes: at least one maximum first derivative *M* is searched for in a current oscillogram; a first point *P3* with the first derivative greater than *k1*M* is searched forward from a diastolic end of the current first derivative *M*; a first point *P4* with the first derivative greater than *k2*M* is searched backward from the point *P3*; the point *P4* is selected as the characteristic point of the current oscillogram; where *0<k2<k1*<1. For example, a value of *k2* ranges from 10% to 20%, and a value of *k1* is 50%.

In the second embodiment, the tangent intersection method is used to obtain the characteristic point in each oscillogram. As shown in FIG. 4, in this embodiment, the method specifically includes: at least one point *P5* with the maximum first derivative is searched for in the current oscillogram, a tangent is made at the point *P5* and is identified as a systolic tangent; a lowest point *P6* of a diastole is searched for in a period corresponding to the point *P5*; a tangent is made at the point *P6* and is identified as a diastolic tangent; an intersection point *P7* of the systolic tangent and diastolic tangent is calculated and is selected as the characteristic point of the current oscillogram.

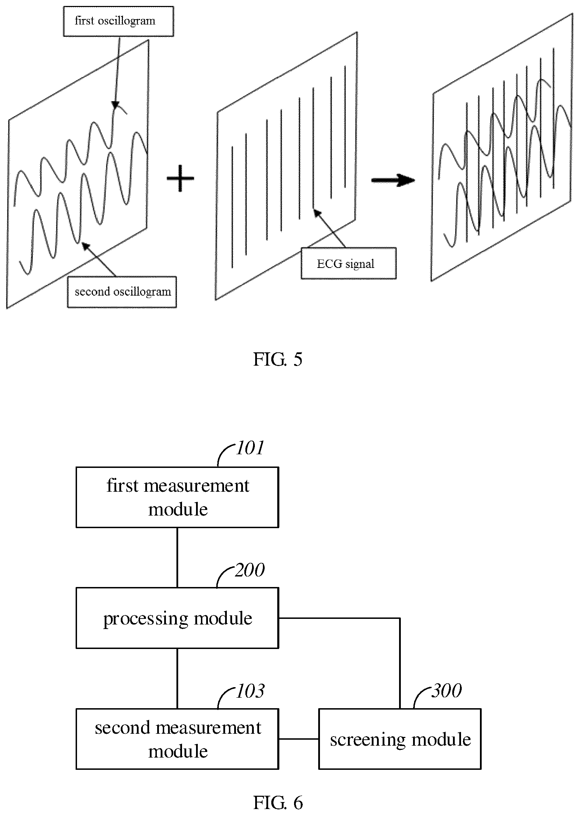

In a specific example of the present application, on a user's PWV function interface, the active cursor is moved to a target ultrasound image by rolling the trackball, with reference to FIG. 5, after a target is selected, the R peaks time data of the ECG signal in the current image and the time data at the (carotid/femoral) arterial blood flow acceleration points are received; the (carotid/femoral) arterial blood flow acceleration points are the characteristic points X and Y selected in the two oscillograms described in the above. Further, the above calculation shows that there is an invalid characteristic point between the selected characteristic points X and *Y*. After screening of these characteristic points, multiple valid cardiac cycles are obtained. It should be noted that in this example, an interval between *R* peaks of adjacent heart rate signals is referred to as a cardiac cycle. In the process of screening the valid cardiac cycles, the valid cardiac cycles and invalid cardiac cycles in the oscillogram can be identified by using different colors and/or different marking lines, which is convenient for users to observe. Further, when the screening is completed, the invalid cardiac cycles can be deleted, restored, etc., which is not further detailed here. Finally, the pulse wave velocity (PWV) is obtained by means of the distance *L* between the two measurement points obtained by the system and the measurement time difference *ΔT* obtained by the above process, and finally the arteriosclerosis index *D* is obtained by means of an equation *D=(3.57*/*PWV)².*

As shown in FIG. 6, in an embodiment of the present application, a measurement system for obtaining an arteriosclerosis index based on an ultrasound device. The measurement system includes: a first measurement module 101, a second measurement module 103, a processing module 200, and a screening module 300.

The first measurement module 101 is configured to acquire two measurement points *P1* and *P2* at different measurement positions at random, and record an extension distance *L1* between the two measurement points *P1* and *P2.* The second measurement module 103 is configured to obtain a measurement time difference *ΔT* between the two measurement points by using an *R* peaks of an ECG signal as a time synchronization signal. The processing module 200 is configured to obtain a pulse wave velocity (PWV) according to the extension distance *L1* and the measurement time difference *ΔT,* with *PWV=L*/Δ*T,* and obtain an arteriosclerosis index *D* with *D*=(3.57/PWV)² according to the pulse wave velocity (PWV).

Different measurement positions usually refer to two blood vessel positions far apart. In the specific embodiment of the present application, the two blood vessel positions can be selected as the carotid artery and the femoral artery respectively, and the two measurement points *P1* and *P2* are any points respectively on the carotid artery and the femoral artery.

After the positions of the two measurement points are selected, the extension distance between the two measurement points can be obtained in various manners. The manner for obtaining the extension distance *L* belongs to the prior art, and will not be further described here. It should be noted that the extension distance *L* is a physical extension distance formed by a flow path of the blood flow from one measurement point to another measurement point.

The second measurement module 103 is specifically configured to: obtain an oscillogram at each measurement point, select at least one characteristic point in the oscillogram corresponding to each measurement point, obtain a time difference between each characteristic point and the *R* peaks of the ECG signal, and obtain *ΔT* according to a plurality of time differences obtained between a plurality of characteristic points and the *R* peaks of the ECG signal.

As shown in FIG. 2, in this example, the time difference between an *R* peaks of the ECG signal and the selected characteristic point in the oscillogram corresponding to one of the measurement points is represented by *t*.

Correspondingly, in this embodiment, the second measurement module 103 is configured to: identify the oscillogram corresponding to the measurement point *P1* as a first oscillogram, identify the oscillogram corresponding to the measurement point *P2* as a second oscillogram, identify a characteristic point selected in the first oscillogram as a characteristic point *X*, identify a characteristic point selected in the second oscillogram as a characteristic point *Y*, identify a difference value between each characteristic point X and an *R* peaks of the ECG signal selected according to the same regulation as *ΔX,* identify a difference value between each characteristic point *Y* and the *R* peaks of the ECG signal selected according to the same regulation as *ΔY,* calculate an average value Δ*X̅* of *ΔX* and an average value Δ*Y̅* of *ΔY,* then obtain Δ*T* =|Δ*X̅* - Δ*Y̅*|.

It should be noted that if the numbers of the selected characteristic identifiers in the first oscillogram and the second oscillogram are both one, there is no need to calculate Δ*X̅* and Δ*Y̅* , but the *ΔT* can be obtained directly by using the Δ*X* and *ΔY* corresponding to each characteristic point. That is, when the number of the selected characteristic identifiers are both one in the two oscillograms, it is satisfied that *ΔT=*|*ΔX-ΔY*|*.*

According to the claimed invention, when multiple characteristic points are selected in each oscillogram, there may exist an abnormal parameter in the multiple characteristic points. In the preferred embodiment of the present application, in order to ensure the accuracy of calculation, the screening module 300 is configured to determine whether an absolute value of the difference value between each *ΔX* and Δ*X̅* is greater than a first preset threshold. If yes, it is determined that the current *ΔX* is invalid. After the invalid *ΔX* is excluded, the second measurement module 103 recalculates Δ*X̅* and ends the calculation when confirming that absolute value of the difference value between each ΔX and the Δ*X̅* obtained by recalculating is not greater than the first preset threshold; the second measurement module 103 determines whether the absolute value of the difference value between each *ΔY* and Δ*Y̅* is greater than the second preset threshold; if yes, it is determined that the current *ΔY* is invalid; after the invalid *ΔY* is excluded, the second measurement module 103 recalculates the Δ*Y̅* and the ends the calculation when confirming that the absolute value of the difference value between each *ΔY* and the Δ*Y̅* obtained by recalculating is not greater than the second preset threshold.

The first preset threshold and the second preset threshold are both a set known parameter value, and the magnitudes thereof are related to the heart rate signal, for example, 0.1s, which will not be further described here.

In an implementation manner of the present application, there are two manners to select the characteristic points.

In a first implementation manner, a derivative threshold method is utilized to acquire a characteristic point in each oscillogram. As shown in FIG. 3, in this implementation manner, the second measurement module 103 is configured to search for at least one maximum first derivative *M* in the current oscillogram, search forward a first point *P3* with the first derivative greater than *k1*M* from a diastolic end of the current first derivative *M,* search backward a first point *P4* with the first derivative greater than *k2*M* from the point *P3,* and select the point *P4* as the characteristic point of the current oscillogram; where *0<k2<k1*<1*.* For example, a value of *k2* ranges from 10% to 20%, and a value of *k1* is 50%.

In a second implementation manner, a tangent intersection method is utilized to obtain the characteristic point in each oscillogram. As shown in FIG. 4, in this implementation manner, the second measurement module 103 is configured to search for at least one point *P5* with the maximum first derivative in the current oscillogram, make a tangent at the point *P5* and identify the tangent as a systolic tangent, search for a lowest point *P6* of a diastole in a period corresponding to the point *P5,* make a tangent at the point *P6* and identify the tangent as a diastolic tangent, calculate an intersection point *P7* of the systolic tangent and diastolic tangent and select the intersection point *P7* as the characteristic point of the current oscillogram.

Those skilled in the art can clearly understand that, for the convenience and conciseness of the description, the specific working process of the measurement system described above can refer to the corresponding process in the foregoing method implementation, which will not be repeated here.

In view of this, in the measurement method and system for obtaining an arteriosclerosis index based on an ultrasound device of the present application, the *R* peaks of the ECG signal is taken as a time synchronization signal to calculate the measurement time difference between two measurement points to obtain the arteriosclerosis index. Accordingly, the calculation accuracy is improved and the measurement cost is reduced without introducing other devices.

In the several implementation manners provided in this application, it should be appreciated that the disclosed measurement system, system and method can be implemented in other ways. For example, the implementation manner of the measurement system described above is just exemplary. For example, the division of the modules is only a logical function division, and there may be other divisions in actual implementation, for example, multiple modules or components can be combined or integrated into another system, or some features can be ignored or not implemented. In addition, the displayed or discussed mutual coupling or direct coupling or communication connection can be indirect coupling or communication connection through some interfaces, systems or modules, and can be in electrical, mechanical or other forms.

The modules described as separate components may or may not be physically separated, and the components displayed as modules may or may not be physical modules, that is, they may be located in one place, or they may be distributed on multiple network modules. Some or all of the modules can be selected according to actual requirements to achieve the objectives of the solution of this embodiment.

Furthermore, function modules in various embodiments of the present application may be integrated into one processing module, or each module may exist alone physically, or two or more modules may be integrated into one module. The above-mentioned integrated modules can be implemented in the form of hardware, or in the form of hardware plus software function modules.

The above-mentioned integrated modules implemented in the form of the software function module may be stored in a computer-readable storage medium. The above-mentioned software function module is stored in a storage medium, and includes a number of instructions to make a computer system (which may be a personal computer, a server, or a network system, etc.) or a processor execute part of the steps of the method described in the various embodiments of the application. The aforementioned storage media can include: a U disk, a mobile hard disk, a Read-Only Memory (ROM), a Random Access Memory (RAM), a magnetic disk or an optical disk and other media that can store program codes.

Finally, it should be noted that the above embodiments are merely used for illustrating the technical solution of the application, rather than limiting it. Although the application has been described in detail with reference to the foregoing embodiments, those of ordinary skill in the art should understand that: the technical solution described in the foregoing embodiments can be modified, or some of the technical features can be replaced; and these modifications or replacements do not cause the technical solution of the present application essentially deviate from the scope as defined by the appended claims.

## Claims

1. A measurement method for obtaining an arteriosclerosis index based on an ultrasound device, **characterized by** comprising:
acquiring (S1) two measurement points *P1* and *P2* at different measurement positions at random, recording an extension distance *L* between the two measurement points *P1* and *P2,* acquiring a measurement time difference *ΔT* between the two measurement points by using an *R* peaks of an ECG signal as a time synchronization signal;
acquiring (S2) a pulse wave velocity, PWV, according to the extension distance L and the measurement time difference *ΔT,* wherein *PWV=L*/Δ*T;*
acquiring (S3) the arteriosclerosis index D according to the PWV, wherein *D*=(3.57/PWV)²;
wherein the acquiring the measurement time difference *ΔT* between the two measurement points by using the *R* peaks of an ECG signal as a time synchronization signal specifically comprises:
acquiring an oscillogram at each measurement point, and selecting at least one characteristic point in the oscillogram corresponding to the each measurement point;
acquiring a time difference between each characteristic point and the *R* peaks of the ECG signal;
obtaining the *ΔT* according to a plurality of time difference obtained between a plurality of characteristic points and the *R* peaks of the ECG signal;
the measurement method further comprising:
identifying an oscillogram corresponding to the measurement point *P1* as a first oscillogram, and identifying an oscillogram corresponding to the measurement point *P2* as a second oscillogram;
identifying a characteristic point selected in the first oscillogram as a characteristic point *X*, and identifying a characteristic point selected in the second oscillogram as a characteristic point *Y*;
identifying a difference value between each characteristic point *X* and the *R* peaks of the ECG signal selected according to a same regulation as *ΔX,* and identifying a difference value between each characteristic point *Y* and the *R* peaks of the ECG signal selected according to the same regulation as *ΔY;*
calculating an average value Δ*X̅* of *ΔX* and an average value Δ*Y̅* of *ΔY,* and obtaining Δ*T* = |Δ*X̅* - Δ*Y̅*|;
the measurement method further comprising:
determining whether an absolute value of a difference value between each *ΔX* and Δ*X̅* is greater than a first preset threshold, determining that a current *ΔX* is invalid if the absolute value of the difference value between each *ΔX* and Δ*X̅* is greater than the first preset threshold, recalculating the Δ*X̅* after excluding the invalid *ΔX,* and ending the calculation when determining that the absolute value of the difference value between each *ΔX* and the Δ*X̅* obtained by recalculating is not greater than the first preset threshold;
determining whether an absolute value of a difference value between each Δ*Y* and Δ*Y̅* is greater than a second preset threshold, determining that a current Δ*Y* is invalid if the absolute value of the difference value between each Δ*Y* and Δ*Y̅* is greater than the second preset threshold, recalculating the Δ*Y̅* after excluding the invalid Δ*Y,* and ending the calculation when determining that the absolute value of the difference value between each Δ*Y* and the Δ*Y̅* obtained by recalculating is not greater than the second preset threshold.

2. The measurement method for obtaining the arteriosclerosis index based on the ultrasonic device according to claim 1, wherein "the selecting at least one characteristic point in the oscillogram corresponding to the each measurement point" specifically comprises:
searching for at least one maximum first derivative *M* in a current oscillogram, searching forward a first point *P3* with a first derivative greater than *k1*M* from a diastolic end of the current first derivative *M,* searching backward a first point *P4* with a first derivative greater than *k2*M* from the point *P3,* and selecting the point *P4* as the characteristic point of the current oscillogram, wherein *0<k2<k1*<1;
or,
searching for at least one point *P5* with a maximum first derivative in the current oscillogram, making a tangent at the point *P5* and identifying the tangent as a systolic tangent, searching for a lowest point *P6* of a diastole in a period corresponding to the point *P5,* making a tangent at the point *P6* and identifying the tangent as a diastolic tangent, calculating an intersection point *P7* of the systolic tangent and diastolic tangent, and selecting the intersection point *P7* as the characteristic point of the current oscillogram.

3. A measurement system for obtaining an arteriosclerosis index based on an ultrasound device, **characterized by** comprising:
a first measurement module (101), configured to acquire two measurement points *P1* and *P2* at different measurement positions at random, and record an extension distance *L1* between the two measurement points *P1* and *P2*;
a second measurement module (103), configured to obtain a measurement time difference *ΔT* between the two measurement points by using an *R* peaks of an ECG signal as a time synchronization signal;
a processing module (200), configured to obtain a pulse wave velocity, PWV, according to the extension distance *L1* and the measurement time difference *ΔT,* wherein *PWV=L*/Δ*T,* and acquiring the arteriosclerosis index *D* according to the pulse wave velocity (PWV), wherein *D*=(3.57/PWV)²;
wherein the second measurement module (103) is specifically configured to: acquire an oscillogram at each measurement point, select at least one characteristic point in the oscillogram corresponding to each measurement point;
acquire a time difference between each characteristic point and the *R* peaks of the ECG signal; and
obtain *ΔT* according to a plurality of time differences obtained between a plurality of characteristic points and the *R* peaks of the ECG signal;
wherein the second measurement module (103) is specifically configured to: identify an oscillogram corresponding to the measurement point *P1* as a first oscillogram, identify an oscillogram corresponding to the measurement point *P2* as a second oscillogram;
identify a characteristic point selected in the first oscillogram as a characteristic point *X*, identify a characteristic point selected in the second oscillogram as a characteristic point *Y*;
identify a difference value between each characteristic point *X* and an *R* peaks of the ECG signal selected according to a same regulation as *ΔX,* identify a difference value between each characteristic point *Y* and the *R* peaks of the ECG signal selected according to the same regulation as Δ*Y;* and
calculate an average value Δ*X̅* of the *ΔX* and an average value Δ*Y̅* of the *ΔY,* and obtain Δ*T* = |Δ*X̅* - Δ*Y̅*|;
the measurement system further comprising a screening module (300) configured to: determine whether an absolute value of a difference value between each *ΔX* and Δ*X̅* is greater than a first preset threshold, determine that a current *ΔX* is invalid if the absolute value of the difference value between each *ΔX* and Δ*X̅* is greater than the first preset threshold, recalculate the Δ*X̅* after excluding the invalid *ΔX,* and end the calculation when determining that the absolute value of the difference value between each ΔX and the Δ*X̅* obtained by recalculating is not greater than the first preset threshold;
determine whether an absolute value of a difference value between each *ΔY* and Δ*Y̅* is greater than a second preset threshold, determine that a current *ΔY* is invalid when the absolute value of the difference value between each *ΔY* and the Δ*Y̅* is greater than the second preset threshold, recalculate the Δ*Y̅* after excluding the invalid *ΔY,* and end the calculation when determining that the absolute value of the difference value between each *ΔY* and the Δ*Y̅* obtained by recalculating is not greater than the second preset threshold.

4. The measurement system for obtaining the arteriosclerosis index based on the ultrasonic device according to claim 3, wherein the second measurement module (103) is configured to, when selecting at least one characteristic point in the oscillogram corresponding to each measurement point: search for at least one maximum first derivative *M* in a current oscillogram, search forward a first point *P3* with a first derivative greater than *k1*M* from a diastolic end of the current first derivative *M,* search backward a first point *P4* with a first derivative greater than *k2*M* from the point *P3,* and select the point *P4* as the characteristic point of the current oscillogram, wherein *0<k2<k1<* 1;
or,
search for at least one point *P5* with a maximum first derivative in the current oscillogram, make a tangent at the point *P5* and identify the tangent as a systolic tangent, search for a lowest point *P6* of a diastole in a period corresponding to the point *P5,* make a tangent at the point *P6* and identify the tangent as a diastolic tangent, calculate an intersection point *P7* of the systolic tangent and diastolic tangent, and select the intersection point *P7* as the characteristic point of the current oscillogram.

## Patentansprüche

1. Messverfahren zum Erhalten eines Arteriosklerose-Index basierend auf einer Ultraschallvorrichtung, **dadurch gekennzeichnet, dass** es umfasst:
zufälliges Erfassen (S1) zweier Messpunkte *P1* und *P2* an unterschiedlichen Messpositionen, Aufzeichnen einer Ausdehnungsdistanz *L* zwischen den zwei Messpunkten *P1* und *P2*, Erfassen einer Messzeitdifferenz *ΔT* zwischen den zwei Messpunkten durch Verwenden einer R-Zacke eines EKG-Signals als ein Zeitsynchronisationssignal;
Erfassen (S2) einer Pulswellengeschwindigkeit, PWV, gemäß der Ausdehnungsdistanz *L* und der Messzeitdifferenz *ΔT,* wobei *PWV=L*/Δ*T,*
Erfassen (S3) des Arteriosklerose-Index *D* gemäß der PWV, wobei *D*=(3,57/PWV)²;
wobei das Erfassen der Messzeitdifferenz *ΔT* zwischen den zwei Messpunkten durch Verwenden der *R*-Zacken eines EKG-Signals als ein Zeitsynchronisationssignal spezifisch umfasst:
Erfassen eines Oszillogramms an jedem Messpunkt und Auswählen mindestens eines charakteristischen Punkts in dem Oszillogramm, das dem jeweiligen Messpunkt entspricht;
Erfassen einer Zeitdifferenz zwischen jedem charakteristischen Punkt und den *R*-Zacken des EKG-Signals;
Erhalten der *ΔT* gemäß einer Vielzahl von Zeitdifferenzen, die zwischen einer Vielzahl von charakteristischen Punkten und den *R*-Zacken des EKG-Signals erhalten werden;
das Messverfahren ferner umfassend:
Identifizieren eines Oszillogramms, das dem Messpunkt *P1* entspricht, als ein erstes Oszillogramm und Identifizieren eines Oszillogramms, das dem Messpunkt *P2* entspricht, als ein zweites Oszillogramm;
Identifizieren eines charakteristischen Punkts, der in dem ersten Oszillogramm ausgewählt ist, als einen charakteristischen Punkt *X*, und Identifizieren eines charakteristischen Punkts, der in dem zweiten Oszillogramm ausgewählt ist, als einen charakteristischen Punkt *Y*;
Identifizieren eines Differenzwerts zwischen jedem charakteristischen Punkt *X* und den *R*-Zacken des EKG-Signals, der gemäß einer gleichen Vorschrift als *ΔX* ausgewählt wird, und Identifizieren eines Differenzwerts zwischen jedem charakteristischen Punkt *Y* und den *R*-Zacken des EKG-Signals, der gemäß der gleichen Vorschrift als *ΔY* ausgewählt wird;
Berechnen eines Durchschnittswerts Δ*X̅* von *ΔX* und eines Durchschnittswerts von *ΔY,* und Erhalten von Δ*T =* |ΔX̅ -Δ*Y̅*|;
das Messverfahren ferner umfassend:
Bestimmen, ob ein Absolutwert eines Differenzwerts zwischen jedem *ΔX* und Δ*X̅* größer als eine erste voreingestellte Schwelle ist, Bestimmen, dass ein aktuelles *ΔX* ungültig ist, falls der Absolutwert des Differenzwerts zwischen jedem *ΔX* und Δ*X̅* größer als die erste voreingestellte Schwelle ist, Neuberechnen des Δ*X̅* nach Ausschließen des ungültigen *ΔX,* und Beenden der Berechnung, wenn bestimmt wird, dass der Absolutwert des Differenzwerts zwischen jedem *ΔX* und dem Δ*X̅* , der durch Neuberechnen erhalten wird, nicht größer als die erste voreingestellte Schwelle ist;
Bestimmen, ob ein Absolutwert eines Differenzwerts zwischen jedem *ΔY* und ΔY̅ größer als eine zweite voreingestellte Schwelle ist, Bestimmen, dass ein aktuelles *ΔY* ungültig ist, falls der Absolutwert des Differenzwerts zwischen jedem Δ*Y* und Δ*Y̅* größer als die zweite voreingestellte Schwelle ist, Neuberechnen des Δ*Y̅* nach Ausschließen des ungültigen *ΔY,* und Beenden der Berechnung, wenn bestimmt wird, dass der Absolutwert des Differenzwerts zwischen jedem *ΔY* und dem Δ*Y̅*, der durch Neuberechnen erhalten wird, nicht größer als die zweite voreingestellte Schwelle ist.

2. Messverfahren zum Erhalten des Arteriosklerose-Index basierend auf der Ultraschallvorrichtung nach Anspruch 1, wobei "das Auswählen mindestens eines charakteristischen Punkts in dem Oszillogramm, das jedem Messpunkt entspricht," spezifisch umfasst:
Suchen nach mindestens einem maximalen ersten Derivat *M* in einem aktuellen Oszillogramm, Vorwärtssuchen nach einem ersten Punkt *P3* mit einem ersten Derivat größer als *k1*M* von einem diastolischen Ende des aktuellen ersten Derivats *M* ausgehend, Rückwärtssuchen nach einem ersten Punkt *P4* mit einem ersten Derivat größer als *k2*M* von dem Punkt *P3* ausgehend, und Auswählen des Punkts *P4* als den charakteristischen Punkt des aktuellen Oszillogramms, wobei *0<k2<k1*<1;
oder,
Suchen nach mindestens einem Punkt *P5* mit einem maximalen ersten Derivat in dem aktuellen Oszillogramm, Anlegen einer Tangente an dem Punkt *P5* und Identifizieren der Tangente als eine systolische Tangente, Suchen nach einem tiefsten Punkt *P6* einer Diastole in einer Periode, die dem Punkt *P5* entspricht, Anlegen einer Tangente an dem Punkt *P6* und Identifizieren der Tangente als eine diastolische Tangente, Berechnen eines Schnittpunkts *P7* der systolischen Tangente und der diastolischen Tangente und Auswählen des Schnittpunkts *P7* als den charakteristischen Punkt des aktuellen Oszillogramms.

3. Messsystem zum Erhalten eines Arteriosklerose-Index basierend auf einer Ultraschallvorrichtung, **dadurch gekennzeichnet, dass** es umfasst:
ein erstes Messmodul (101), das konfiguriert ist, um zwei Messpunkte *P1* und *P2* an unterschiedlichen Messpositionen zufällig zu erfassen und eine Ausdehnungsdistanz *L1* zwischen den zwei Messpunkten *P1* und *P2* aufzuzeichnen;
ein zweites Messmodul (103), das konfiguriert ist, um eine Messzeitdifferenz *ΔT* zwischen den zwei Messpunkten durch Verwenden einer *R*-Zacke eines EKG-Signals als ein Zeitsynchronisationssignal zu erhalten;
ein Verarbeitungsmodul (200), das konfiguriert ist, um eine Pulswellengeschwindigkeit, PWV, gemäß der Ausdehnungsdistanz *L1* und der Messzeitdifferenz *ΔT* zu erhalten, wobei *PWV=L*/Δ*T,* und den Arteriosklerose-Index *D* gemäß der Pulswellengeschwindigkeit (PWV) zu erfassen, wobei *D*=(3,57/PWV)²;
wobei das zweite Messmodul (103) spezifisch konfiguriert ist zum: Erfassen eines Oszillogramms an jedem Messpunkt, Auswählen mindestens eines charakteristischen Punkts in dem Oszillogramm, der jedem Messpunkt entspricht;
Erfassen einer Zeitdifferenz zwischen jedem charakteristischen Punkt und den R-Zacken des EKG-Signals; und
Erhalten von *ΔT* gemäß einer Vielzahl von Zeitdifferenzen, die zwischen einer Vielzahl von charakteristischen Punkten und den R-Zacken des EKG-Signals erhalten werden;
wobei das zweite Messmodul (103) spezifisch konfiguriert ist zum: Identifizieren eines Oszillogramms, das dem Messpunkt *P1* entspricht, als ein erstes Oszillogramm, Identifizieren eines Oszillogramms, das dem Messpunkt *P2* entspricht, als ein zweites Oszillogramm;
Identifizieren eines charakteristischen Punkts, der in dem ersten Oszillogramm ausgewählt ist, als einen charakteristischen Punkt X, Identifizieren eines charakteristischen Punkts, der in dem zweiten Oszillogramm ausgewählt ist, als einen charakteristischen Punkt *Y*;
Identifizieren eines Differenzwerts zwischen jedem charakteristischen Punkt X und den *R*-Zacken des EKG-Signals, der gemäß einer gleichen Vorschrift als *ΔX* ausgewählt wird, und Identifizieren eines Differenzwerts zwischen jedem charakteristischen Punkt *Y* und den *R*-Zacken des EKG-Signals, der gemäß der gleichen Vorschrift als *ΔY* ausgewählt wird; und
Berechnen eines Durchschnittswerts Δ*X̅* der *ΔX* und eines Durchschnittswerts Δ*Y̅* der *ΔY,* und Erhalten von Δ*T* = |Δ*X̅* -Δ*Y̅*|;
das Messsystem ferner umfassend ein Prüfmodul (300), das konfiguriert ist zum: Bestimmen, ob ein Absolutwert eines Differenzwerts zwischen jedem *ΔX* und Δ*X̅* größer als eine erste voreingestellte Schwelle ist, Bestimmen, dass ein aktuelles *ΔX* ungültig ist, falls der Absolutwert des Differenzwerts zwischen jedem *ΔX* und Δ*X̅* größer als die erste voreingestellte Schwelle ist, Neuberechnen des Δ*X̅* nach Ausschließen des ungültigen *ΔX,* und Beenden der Berechnung, wenn bestimmt wird, dass der Absolutwert des Differenzwerts zwischen jedem *ΔX* und dem Δ*X̅* , der durch Neuberechnen erhalten wird, nicht größer als die erste voreingestellte Schwelle ist;
Bestimmen, ob ein Absolutwert eines Differenzwerts zwischen jedem *ΔY* und Δ*Y̅* größer als eine zweite voreingestellte Schwelle ist, Bestimmen, dass ein aktuelles *ΔY* ungültig ist, wenn der Absolutwert des Differenzwerts zwischen jedem *ΔY* und dem Δ*Y̅* größer als die zweite voreingestellte Schwelle ist, Neuberechnen des Δ*Y̅* nach Ausschließen des ungültigen *ΔY,* und Beenden der Berechnung, wenn bestimmt wird, dass der Absolutwert des Differenzwerts zwischen jedem *ΔY* und dem Δ*̅Y̅*̅ , der durch Neuberechnen erhalten wird, nicht größer als die zweite voreingestellte Schwelle ist.

4. Messsystem zum Erhalten des Arteriosklerose-Index basierend auf der Ultraschallvorrichtung nach Anspruch 3, wobei das zweite Messmodul (103) konfiguriert ist, um, wenn mindestens ein charakteristischer Punkt in dem Oszillogramm ausgewählt wird, das jedem Messpunkt entspricht: nach mindestens einem maximalen ersten Derivat *M* in einem aktuellen Oszillogramm zu suchen, nach einem ersten Punkt *P3* mit einem ersten Derivat größer als *k1*M* von einem diastolischen Ende des aktuellen ersten Derivats *M* ausgehend vorwärts zu suchen, nach einem ersten Punkt *P4* mit einem ersten Derivat größer als *k2*M* von dem Punkt P3 ausgehend rückwärts zu suchen, und den Punkt *P4* als den charakteristische Punkt des aktuellen Oszillogramms auszuwählen, wobei *0<k2<k1*<1;
oder,
nach mindestens einem Punkt *P5* mit einem maximalen ersten Derivat in dem aktuellen Oszillogramm zu suchen, eine Tangente an dem Punkt *P5* anzulegen und die Tangente als eine systolische Tangente zu identifizieren, nach einem tiefsten Punkt *P6* einer Diastole in einer Periode, die dem Punkt *P5* entspricht, zu suchen, eine Tangente an dem Punkt *P6* anzulegen und die Tangente als eine diastolische Tangente zu identifizieren, einen Schnittpunkts *P7* der systolischen Tangente und der diastolischen Tangente zu berechnen und den Schnittpunkts *P7* als den charakteristischen Punkt des aktuellen Oszillogramms auszuwählen.

## Revendications

1. Procédé de mesure permettant d'obtenir un indice d'artériosclérose en fonction d'un dispositif à ultrasons, **caractérisé en ce qu'il** comprend :
l'acquisition (S1) de deux points de mesure *P1* et *P2* à différentes positions de mesure au hasard, l'enregistrement d'une distance d'extension *L* entre les deux points de mesure *P1* et *P2,* l'acquisition d'une différence de temps de mesure *ΔT* entre les deux points de mesure en utilisant des pics *R* d'un signal ECG en tant que signal de synchronisation temporelle ;
l'acquisition (S2) d'une vitesse d'onde de pouls, PWV, selon la distance d'extension L et la différence de temps de mesure *ΔT,* dans lequel *PWV=L*/Δ*T,*
l'acquisition (S3) de l'indice d'artériosclérose D selon la PWV, dans lequel *D*=(3,57/PWV)² ;
dans lequel l'acquisition de la différence de temps de mesure *ΔT* entre les deux points de mesure en utilisant les pics *R* d'un signal ECG en tant que signal de synchronisation temporelle comprend spécifiquement :
l'acquisition d'un oscillogramme à chaque point de mesure, et la sélection d'au moins un point caractéristique dans l'oscillogramme correspondant à chaque point de mesure précité ;
l'acquisition d'une différence de temps entre chaque point caractéristique et les pics *R* du signal ECG ;
l'obtention du *ΔT* selon une pluralité de différences de temps obtenues entre une pluralité de points caractéristiques et les pics *R* du signal ECG ;
le procédé de mesure comprenant en outre :
l'identification d'un oscillogramme correspondant au point de mesure *P1* en tant que premier oscillogramme, et l'identification d'un oscillogramme correspondant au point de mesure *P2* en tant que second oscillogramme ;
l'identification d'un point caractéristique sélectionné dans le premier oscillogramme en tant que point caractéristique *X*, et l'identification d'un point caractéristique sélectionné dans le second oscillogramme en tant que point caractéristique *Y*;
l'identification d'une valeur de différence entre chaque point caractéristique *X* et les pics *R* du signal ECG sélectionnés selon une même règle que *ΔX,* et l'identification d'une valeur de différence entre chaque point caractéristique *Y* et les pics *R* du signal ECG sélectionnés selon la même règle que *ΔY;*
le calcul d'une valeur moyenne Δ*X̅* de *ΔX* et d'une valeur moyenne Δ*Y̅* de *ΔY,* et l'obtention de Δ*T =* |*ΔX̅ - ΔY̅*| ;
le procédé de mesure comprenant en outre :
le fait de déterminer si une valeur absolue d'une valeur de différence entre chaque *ΔX* et Δ*X̅* est supérieure à un premier seuil prédéfini, la détermination qu'un *ΔX* actuel est non valide si la valeur absolue de la valeur de différence entre chaque *ΔX* et Δ*X̅* est supérieure au premier seuil prédéfini, le recalcul du Δ*X̅* après exclusion du *ΔX* non valide, et la fin du calcul lors de la détermination que la valeur absolue de la valeur de différence entre chaque *ΔX* et le Δ*X̅* obtenu par recalcul n'est pas supérieure au premier seuil prédéfini ;
le fait de déterminer si une valeur absolue d'une valeur de différence entre chaque *ΔY* et Δ*Y̅* est supérieure à un second seuil prédéfini, la détermination qu'un *ΔY* actuel est non valide si la valeur absolue de la valeur de différence entre chaque *ΔY* et *ΔY* est supérieure au second seuil prédéfini, le recalcul du Δ*Y̅* après exclusion du *ΔY* non valide, et la fin du calcul lors de la détermination que la valeur absolue de la valeur de différence entre chaque *ΔY* et le Δ*̅Y̅*̅ obtenu par recalcul n'est pas supérieure au second seuil prédéfini.

2. Procédé de mesure permettant d'obtenir l'indice d'artériosclérose en fonction du dispositif à ultrasons selon la revendication 1, dans lequel « la sélection d'au moins un point caractéristique dans l'oscillogramme correspondant à chaque point de mesure précité » comprend spécifiquement :
la recherche d'au moins une dérivée première maximale *M* dans un oscillogramme actuel, la recherche vers l'avant d'un premier point *P3* avec une dérivée première supérieure à *k1*M* à partir d'une extrémité diastolique de la dérivée première actuelle *M,* la recherche vers l'arrière d'un premier point *P4* avec une dérivée première supérieure à *k2*M* à partir du point *P3,* et la sélection du point *P4* en guise de point caractéristique de l'oscillogramme actuel, dans lequel *0<k2<k1*<1 ;
ou,
la recherche d'au moins un point *P5* avec une dérivée première maximale dans l'oscillogramme actuel, le tracé d'une tangente au point *P5* et l'identification de la tangente en tant que tangente systolique, la recherche d'un point le plus bas *P6* d'une diastole dans une période correspondant au point *P5,* le tracé d'une tangente au point *P6* et l'identification de la tangente en tant que tangente diastolique, le calcul d'un point d'intersection *P7* de la tangente systolique et de la tangente diastolique, et la sélection du point d'intersection *P7* en guise de point caractéristique de l'oscillogramme actuel.

3. Système de mesure permettant d'obtenir un indice d'artériosclérose en fonction d'un dispositif à ultrasons, **caractérisé en ce qu'il** comprend :
un premier module de mesure (101), configuré pour acquérir deux points de mesure *P1* et *P2* à différentes positions de mesure au hasard, et enregistrer une distance d'extension *L1* entre les deux points de mesure *P1* et *P2* ;
un second module de mesure (103), configuré pour obtenir une différence de temps de mesure *ΔT* entre les deux points de mesure en utilisant des pics *R* d'un signal ECG en tant que signal de synchronisation temporelle ;
un module de traitement (200), configuré pour obtenir une vitesse d'onde de pouls, PWV, selon la distance d'extension *L1* et la différence de temps de mesure *ΔT,* dans lequel *PWV=L*/Δ*T,* et l'acquisition de l'indice d'artériosclérose *D* selon la vitesse d'onde de pouls (PWV), dans lequel *D*=(3,57/PWV)² ;
dans lequel le second module de mesure (103) est spécifiquement configuré pour : acquérir un oscillogramme à chaque point de mesure, sélectionner au moins un point caractéristique dans l'oscillogramme correspondant à chaque point de mesure ;
acquérir une différence de temps entre chaque point caractéristique et les pics R du signal ECG ; et
obtenir le *ΔT* selon une pluralité de différences de temps obtenues entre une pluralité de points caractéristiques et les pics R du signal ECG ;
dans lequel le second module de mesure (103) est spécifiquement configuré pour : identifier un oscillogramme correspondant au point de mesure *P1* en tant que premier oscillogramme, identifier un oscillogramme correspondant au point de mesure *P2* en tant que second oscillogramme ;
identifier un point caractéristique sélectionné dans le premier oscillogramme en tant que point caractéristique X, et identifier un point caractéristique sélectionné dans le second oscillogramme en tant que point caractéristique Y;
identifier une valeur de différence entre chaque point caractéristique X et un pic *R* du signal ECG sélectionné selon une même règle que *ΔX,* identifier une valeur de différence entre chaque point caractéristique *Y* et les pics *R* du signal ECG sélectionnés selon la même règle que *ΔY* ; et
calculer une valeur moyenne Δ*X̅* du *Δ*X et une valeur moyenne Δ*Y̅* du *ΔY,* et obtenir Δ*T =* |Δ*X̅ -* Δ*Y̅*| ;
le système de mesure comprenant en outre un module de criblage (300) configuré pour : déterminer si une valeur absolue d'une valeur de différence entre chaque *ΔX* et Δ*X̅* est supérieure à un premier seuil prédéfini, déterminer qu'un *ΔX* actuel est non valide si la valeur absolue de la valeur de différence entre chaque *ΔX* et Δ*X̅* est supérieure au premier seuil prédéfini, recalculer le Δ*X̅* après exclusion du *ΔX* non valide, et mettre fin au calcul lors de la détermination que la valeur absolue de la valeur de différence entre chaque *ΔX* et le Δ*X̅* obtenu par recalcul n'est pas supérieure au premier seuil prédéfini ;
déterminer si une valeur absolue d'une valeur de différence entre chaque *ΔY* et Δ*Y̅* est supérieure à un second seuil prédéfini, déterminer qu'un *ΔY* actuel est non valide lorsque la valeur absolue de la valeur de différence entre chaque *ΔY* et le Δ*̅Y̅*̅ est supérieure au second seuil prédéfini, recalculer le Δ*Y̅* après exclusion du *ΔY* non valide, et mettre fin au calcul lors de la détermination que la valeur absolue de la valeur de différence entre chaque *ΔY* et le Δ*Y̅* obtenu par recalcul n'est pas supérieure au second seuil prédéfini.

4. Système de mesure permettant d'obtenir l'indice d'artériosclérose en fonction du dispositif à ultrasons selon la revendication 3, dans lequel le second module de mesure (103) est configuré pour, lors de la sélection d'au moins un point caractéristique dans l'oscillogramme correspondant à chaque point de mesure : rechercher au moins une dérivée première maximale *M* dans un oscillogramme actuel, rechercher vers l'avant un premier point *P3* avec une dérivée première supérieure à *k1*M* à partir d'une extrémité diastolique de la dérivée première *M* actuelle, rechercher vers l'arrière un premier point *P4* avec une dérivée première supérieure à *k2*M* à partir du point *P3,* et sélectionner le point *P4* en guise de point caractéristique de l'oscillogramme actuel, dans lequel *0<k2<k1*<1;
ou,
rechercher au moins un point *P5* avec une dérivée première maximale dans l'oscillogramme actuel, tracer une tangente au point *P5* et identifier la tangente en tant que tangente systolique, rechercher un point le plus bas *P6* d'une diastole dans une période correspondant au point *P5,* tracer une tangente au point *P6* et identifier la tangente en tant que tangente diastolique, calculer un point d'intersection *P7* de la tangente systolique et de la tangente diastolique, et sélectionner le point d'intersection *P7* en guise de point caractéristique de l'oscillogramme actuel.
